# EUROPÄISCHE PATENTANMELDUNG

(11) **EP 4 737 556 A1**
(43) Veröffentlichungstag der Anmeldung: **06.05.2026**
(21) Anmeldenummer: 24209818.4
(22) Anmeldetag: 30.10.2024
(51) Int. Cl.: C12M 3/06, C12M 1/32, C12M 1/00, C12M 1/12

(54) **MIKROFLUIDISCHE PROBENVORRICHTUNGEN UND VERFAHREN ZUR HERSTELLUNG EINER MIKROFLUIDISCHEN UNTERSUCHUNGSVORRICHTUNG**

(71) Anmelder: ibidi GmbH, 82166 Gräfelfing (DE)
(72) Erfinder: Horn, Elias, 90537 Feucht (DE); Zantl, Roman, 82166 Gräfelfing (DE)
(74) Vertreter: Grünecker Patent- und Rechtsanwälte PartG mbB

(57) **Zusammenfassung**

Die Erfindung betrifft eine mikrofluidische Probenvorrichtung, die einen ersten Behälter, der an seiner Unterseite eine erste Öffnung in einer ersten Bodenebene aufweist, und einen zweiten Behälter, der an seiner Unterseite eine zweite Öffnung in einer zweiten Bodenebene aufweist, umfasst, wobei der erste Behälter zumindest teilweise im Inneren des zweiten Behälters angeordnet ist, wobei der erste Behälter mit dem zweiten Behälter verbunden ist und wobei die erste Bodenebene gegenüber der zweiten Bodenebene nach oben versetzt ist.

Die Erfindung betrifft weiterhin eine mikrofluidische Probenvorrichtung, die einen ersten Behälter, der an seiner Unterseite eine erste Öffnung in einer ersten Bodenebene aufweist, und einen zweiten Behälter, der einen geschlossenen Boden aufweist, umfasst, wobei der erste Behälter zumindest teilweise im Inneren des zweiten Behälters angeordnet ist, wobei der erste Behälter mit dem zweiten Behälter verbunden ist und wobei die erste Bodenebene gegenüber dem Boden nach oben versetzt ist.

Die Erfindung betrifft außerdem ein Verfahren zur Herstellung einer mikrofluidischen Untersuchungsvorrichtung, insbesondere der zuvor genannten Probenvorrichtungen, für Untersuchungen.

## Beschreibung

Die Erfindung betrifft mikrofluidische Probenvorrichtungen sowie ein Verfahren zur Herstellung einer mikrofluidischen Untersuchungsvorrichtung.

Im Bereich der Zell- und Gewebekulturmodelle werden biologische Grenzflächen üblicherweise mit Hilfe von porösen und permeablen Membranen erzeugt. Diese Membranen stabilisieren die Zellen in der Kultur und erlauben die Nutzung verschiedener Flüssigkeiten auf beiden Seiten der Membran. Dazu wird ein Einsatz, auf dem eine Membran gespannt ist, in einen Behälter einer Zellkulturplatte oder in eine Petrischale gehängt. Der Einsatz selbst wird befüllt, ebenso der Behälter, so dass die Membran die beiden Flüssigkeiten trennt. Werden Zellen auf dieser Membran kultiviert, können sie auf dieser Grenzfläche kultiviert und untersucht werden. Beide Membranseiten können mit unterschiedlichen Flüssigkeiten oder Konzentrationen gefüllt werden.

Diese Membraneinsätze können biologische Grenzflächen simulieren, also z.B. jene Übergangsbereiche zwischen Geweben im menschlichen Körper, die klar voneinander abgetrennt sind. Typische Zellmodellsysteme, die Grenzflächen bilden und sich in ihrer natürlichen Umgebung abgrenzen, sind z.B. Endothelzellen, Epithelzellen und Nierenzellen.

Kommerziell erhältliche Membraneinsätze sind z.B. Corning^{®}Transwell^{®}, Nunc^{™} Polycarbonat-Zellkultureinsätze oder ThinCert^{®} Zellkultureinsätze (Greiner Bio-One), mit Membranen bestehend aus verschiedenen thermoplastischen Polymerwerkstoffen, wie z.B. Polyester und Polycarbonat. Dabei finden Porengrößen von ca. 0,5 µm bis ca. 12 µm Verwendung.

Derartige herkömmliche Membraneinsätze haben den Nachteil, dass die biologische Grenzfläche nicht nahe an der natürlichen Beschaffenheit nachgebildet wird und das Membranmaterial mikroskopische Untersuchungen durch hohe Autofluoreszenz, schlechte Transmission und Streueffekte erschwert. Es besteht daher ein Bedarf an einer mikrofluidischen Probenvorrichtung, mit der Mikroskopie an einer biologischen Grenzfläche nahe an der natürlichen Beschaffenheit durchgeführt werden kann.

Angesichts dessen besteht die der Erfindung zugrundeliegende Aufgabe bereits darin, eine mikrofluidische Probenvorrichtung bereitzustellen, mittels derer Teile der Probe bzw. eines Mediums, das die Probe umgibt, getrennt werden können.

Diese Aufgabe wird durch eine Probenvorrichtung gemäß Anspruch 1 gelöst.

Die Erfindung stellt eine mikrofluidische Probenvorrichtung bereit, die einen ersten Behälter, der an seiner Unterseite eine erste Öffnung in einer ersten Bodenebene aufweist, und einen zweiten Behälter, der an seiner Unterseite eine zweite Öffnung in einer zweiten Bodenebene aufweist, umfasst, wobei der erste Behälter zumindest teilweise im Inneren des zweiten Behälters angeordnet ist, wobei der erste Behälter mit dem zweiten Behälter verbunden ist und wobei die erste Bodenebene gegenüber der zweiten Bodenebene nach oben versetzt ist.

Durch die zwei Behälter und den Versatz der Bodenebenen können Teile der Probe bzw. des Mediums, das die Probe umgibt, getrennt werden.

Sämtliche Ortsangaben wie beispielsweise "Unterseite", "Innere", "oben", "seitlich" beziehen sich auf den bestimmungsgemäßen Gebrauch der Probenvorrichtung.

Der erste Behälter kann gegenüber dem zweiten Behälter fixiert sein. Das bedeutet, dass der erste Behälter mit dem zweiten Behälter in einer festen Ortsbeziehung steht bzw. gegenüber dem zweiten Behälter eine feste Relativposition einnimmt.

Dass die erste Bodenebene gegenüber der zweiten Bodenebene nach oben versetzt ist, ist gleichbedeutend damit, dass die Unterseite des ersten Behälters gegenüber der Unterseite des zweiten Behälters nach oben versetzt ist. Mit anderen Worten endet der zweite Behälter im bestimmungsgemäßen Gebrauch unterhalb des ersten Behälters.

Die Probenvorrichtung kann ein Bodenelement, das die zweite Öffnung verschließt, umfassen.

Durch das Bodenelement ist der zweite Behälter nach unten geschlossen, wodurch ein Verlust an Flüssigkeit verhindert wird.

Das Bodenelement kann einen Boden des zweiten Behälters bilden. Das Bodenelement kann selbst undurchlässig für Flüssigkeit sein.

Die Probenvorrichtung kann einen dritten Behälter, der einen geschlossenen Boden aufweist, umfassen, wobei der erste Behälter und der zweite Behälter zumindest teilweise im Inneren des dritten Behälters angeordnet sein können.

Durch den dritten Behälter wird ein Auslaufen von Flüssigkeit aus dem ersten und/oder zweiten Behälter vermieden. Der dritte Behälter dient als Reservoir für Flüssigkeit.

An dem geschlossenen Boden kann keine Flüssigkeit austreten.

Der dritte Behälter kann einen Boden des zweiten Behälters bilden.

Der dritte Behälter kann als Vertiefung gemäß Standards von Mikrotiterplatten (z.B. ANSI-Standard "ANSI SLAS 4-2004 (R2012) (formerly recognized as ANSI/SBS 4-2004)") oder Petrischalen ausgebildet sein. Der dritte Behälter kann die Größe eines Objektträgers (z.B. gemäß DIN ISO 8037-1:2003-05) haben.

Ein, sechs, zwölf, 24, 48, 96, 384, und/oder 1536 der jeweils vorgenannten ersten und zweiten Behälter können in einer Mikrotiterplatte, einer Petrischale, und/oder auf einem Objektträger ausgebildet sein.

Der erste Behälter und/oder der zweite Behälter umfassen jeweils eine Seitenwand.

Der erste Behälter kann oberhalb des zweiten Behälters enden. Insbesondere kann die Seitenwand des ersten Behälters oberhalb der Seitenwand des zweiten Behälters enden.

Die Erfindung stellt weiterhin eine mikrofluidische Probenvorrichtung bereit, die einen ersten Behälter, der an seiner Unterseite eine erste Öffnung in einer ersten Bodenebene aufweist, und einen zweiten Behälter, der einen geschlossenen Boden aufweist, umfasst, wobei der erste Behälter zumindest teilweise im Inneren des zweiten Behälters angeordnet ist, wobei der erste Behälter mit dem zweiten Behälter verbunden ist und wobei die erste Bodenebene gegenüber dem Boden nach oben versetzt ist.

Durch die zwei Behälter und den Versatz der Bodenebenen können Teile der Probe bzw. des Mediums, das die Probe umgibt, getrennt werden.

An dem geschlossenen Boden kann keine Flüssigkeit austreten.

Das Bodenelement bzw. der Boden der zuvor beschriebenen Probenvorrichtungen kann ein transparentes Material umfassen oder daraus bestehen.

Durch das transparente Material des Bodenelements bzw. des Bodens wird Mikroskopie durch das Bodenelement bzw. den Boden ermöglicht.

Das transparente Material ist in einem Wellenlängenbereich durchlässig, der für Mikroskopie geeignet ist, insbesondere in einem sichtbaren Wellenlängenbereich des Lichts.

Das transparente Material kann Glas, Cyclo-Olefin-Copolymer (COC), Cyclo-Olefin-Polymer (COP), Polymethylmethacrylat (PMMA), Polycarbonat (PC), Polyethylen (PE) oder Polystyrol (PS) sein. Das Bodenelement kann eine Kunststofffolie umfassen oder daraus bestehen. Die Kunststofffolie kann eine Dicke in einem Bereich von 2 µm bis 0,5 mm, insbesondere eine Dicke von circa 170 µm, haben. Die Kunststofffolie kann eine Kunststofffolie ohne Doppelbrechung sein und/oder mit einer Eigenfluoreszenz, die im Wesentlichen gleich der Eigenfluoreszenz eines herkömmlichen Deckglases ist.

Das Bodenelement bzw. der Boden der zuvor beschriebenen Probenvorrichtungen kann eine Dicke von mindestens 25 µm, insbesondere mindestens 100 µm, insbesondere mindestens 500 µm, und/oder höchstens 2 mm, insbesondere höchstens 1,5 mm, insbesondere höchstens 1 mm, aufweisen. Dabei kann jede der angegebenen Untergrenzen mit jeder der angegebenen Obergrenzen kombiniert werden.

Ein Bodenelement bzw. ein Boden entsprechender Dicke ermöglicht, den Einfluss des Bodenelements bzw. des Bodens auf Mikroskopie, insbesondere hinsichtlich Absorption und Streueffekte, durch das Bodenelement bzw. den Boden gering zu halten und gleichzeitig Stabilität zu gewährleisten.

Die zuvor beschriebenen Probenvorrichtungen können eine Strebe, mittels der der erste Behälter seitlich mit dem zweiten Behälter verbunden ist, umfassen.

Durch das Einfügen der Strebe wird die Stabilität der Probenvorrichtung verbessert.

Die Strebe der zuvor beschriebenen Probenvorrichtungen kann oberhalb der ersten Bodenebene angeordnet sein.

Durch die Strebe oberhalb der ersten Bodenebene wird ermöglicht, dass Flüssigkeit von unten an den ersten Behälter gelangt, da der Raum zwischen der zweiten Bodenebene und der ersten Bodenebene nicht von der Strebe blockiert wird.

Die zuvor beschriebenen Probenvorrichtungen können einstückig oder mehrstückig ausgebildet sein. Beispielsweise können der erste Behälter, der zweite Behälter und die Strebe aus einem Stück bestehen und das Bodenelement kann ein davon getrenntes Stück sein. Das Stück des ersten Behälters, des zweiten Behälters und der Strebe kann im Spritzguss hergestellt sein.

Eine der zuvor beschriebenen Probenvorrichtungen kann einen Abstandshalter, der an der Unterseite des ersten Behälters befestigt ist, umfassen, wobei der Abstandshalter bis zur zweiten Bodenebene bzw. bis zum Boden reicht.

Durch eine entsprechende Probenvorrichtung mit Abstandshalter wird ein Verrutschen des ersten Behälters in vertikaler Richtung verhindert.

Der Abstandshalter kann lediglich in einem Teil umlaufend um einen unteren Rand des ersten Behälters verlaufen.

Der Abstandshalter kann einstückig mit dem ersten Behälter ausgebildet sein.

Der erste Behälter kann über ein weiteres Element mit dem zweiten Behälter verbunden oder an diesem befestigt sein. Das weitere Element kann die Strebe sein oder der Abstandshalter und das Bodenelement bzw. der Boden. Der erste Behälter kann über den Abstandshalter mit dem Bodenelement bzw. dem Boden verbunden sein. Der erste Behälter kann über das Bodenelement bzw. den Boden, insbesondere eine Platte, mit dem zweiten Behälter verbunden sein.

Der zweite Behälter und/oder der Abstandshalter der zuvor beschriebenen Probenvorrichtungen können an ihrer Unterseite eine klebrige Oberfläche aufweisen.

Durch die klebrige Oberfläche kann ein Bodenelement befestigt werden.

"Klebrig" soll in diesem Zusammenhang bedeuten, dass bei Raumtemperatur, insbesondere von 20 bis 25 °C, und 30 - 40 % relativer Luftfeuchtigkeit Klebrigkeit besteht. Insbesondere soll Klebrigkeit beim Verbinden des zweiten Behälters und des Bodenelements bestehen. Die klebrige Verbindung soll 100% flüssigkeitsdicht sein. Die klebrige Oberfläche kann dauerklebrig und/oder nicht-aushärtend ausgebildet sein. Dies ermöglicht zumindest an trockenen Oberflächen ein mehrfaches Verbinden des zweiten Behälters mit dem Bodenelement. Die klebrige Oberfläche kann einen Thermoplasten oder ein Silikon umfassen oder daraus bestehen.

Die Oberfläche kann selbst klebrig sein oder eine klebrige Beschichtung aufweisen.

Eine der zuvor beschriebenen Probenvorrichtungen kann weiterhin ein Hydrogel, das die erste Öffnung verschließt, umfassen.

Durch das Hydrogel wird eine Grenzfläche zwischen dem Inneren des ersten und dem Inneren des zweiten Behälters, insbesondere dem außerhalb des zweiten Behälters befindlichen Inneren des ersten Behälters, erzeugt.

Das Hydrogel kann einen Elastizitätsmodul in einem Bereich von 1 bis 10 kPa aufweisen. Das Hydrogel kann Matrigel, Collagen, Fibrin, Polyacrylamid, GelMA, Agarose oder ein Derivat dergleichen sein. Eine Gelpolymerisierung des Hydrogels kann unter Änderung des pH-Werts des Hydrogels, Temperaturänderung, oder UV-Belichtung erfolgen.

Das Hydrogel kann sich bis zur zweiten Bodenebene bzw. bis zum Bodenelement bzw. Boden erstrecken. Das Hydrogel kann eine Dicke von mindestens 0,05 mm, insbesondere mindestens 0,1 mm, insbesondere mindestens 0,5 mm, insbesondere mindestens 1 mm und/oder höchstens 5 mm, insbesondere höchstens 4,5 mm insbesondere höchstens 4 mm, insbesondere höchstens 3 mm, aufweisen. Dabei kann jede der angegebenen Untergrenzen mit jeder der angegebenen Obergrenzen kombiniert werden.

Durch die mittels des Hydrogels verschlossene erste Öffnung kann durch Diffusion bzw. Fluss aufgrund eines Druckunterschieds Flüssigkeit hindurchtreten.

Der erste Behälter einer der zuvor beschriebenen Probenvorrichtungen kann ein Volumen von mindestens 20 µl, insbesondere mindestens 50 µl, insbesondere mindestens 100 µl, insbesondere mindestens 500 µl, und/oder höchstens 2000 µl, insbesondere höchstens 1500 µl, insbesondere höchstens 1000 µl, haben. Dabei kann jede der angegebenen Untergrenzen mit jeder der angegebenen Obergrenzen kombiniert werden.

Durch ein entsprechendes Volumen kann der erste Behälter in einem mikrofluidischen Aufbau verwendet werden.

Der erste Behälter kann einen kreisförmigen, elliptischen oder rechteckigen Grundriss haben. Der erste Behälter kann zylindrisch sein. Das Volumen des ersten Behälters ist das Volumen des Inneren des ersten Behälters. Der erste Behälter kann einen äußeren Durchmesser von mindestens 2 mm, insbesondere mindestens 3 mm, insbesondere mindestens 4 mm, insbesondere circa 4,5 mm und/oder höchstens 15 mm, insbesondere höchstens 10 mm, insbesondere höchstens 5 mm haben. Dabei kann jede der angegebenen Untergrenzen mit jeder der angegebenen Obergrenzen kombiniert werden.

Der zweite Behälter kann ein Volumen von mindestens 20 µl, insbesondere mindestens 50 µl, insbesondere mindestens 100 µl, insbesondere mindestens 500 µl, und/oder höchstens 2000 µl, insbesondere höchstens 1500 µl, insbesondere höchstens 1000 µl, haben. Dabei kann jede der angegebenen Untergrenzen mit jeder der angegebenen Obergrenzen kombiniert werden.

Der zweite Behälter kann einen kreisförmigen, elliptischen oder rechteckigen Grundriss haben. Der zweite Behälter kann zylindrisch sein. Das Volumen des zweiten Behälters ist das Volumen des Inneren des zweiten Behälters. Der zweite Behälter kann einen äußeren Durchmesser von mindestens 4 mm, insbesondere mindestens 5 mm, insbesondere mindestens 10 mm, insbesondere circa 12 mm und/oder höchstens 50 mm, insbesondere höchstens 25 mm, insbesondere höchstens 15 mm haben. Dabei kann jede der angegebenen Untergrenzen mit jeder der angegebenen Obergrenzen kombiniert werden.

Die erste Öffnung einer der zuvor beschriebenen Probenvorrichtungen kann einen Durchmesser von mindestens 0,5 mm, insbesondere mindestens 2 mm, insbesondere circa 3 mm, und/oder höchstens 8 mm, insbesondere höchstens 6 mm, insbesondere höchstens 4 mm aufweisen. Dabei kann jede der angegebenen Untergrenzen mit jeder der angegebenen Obergrenzen kombiniert werden.

Durch einen entsprechenden Durchmesser der ersten Öffnung wird Mikroskopie in der ersten Öffnung ohne störenden Einfluss des ersten Behälters ermöglicht.

Eine der zuvor beschriebenen Probenvorrichtungen kann ein thermoplastisches Polymer und/oder ein Elastomer umfassen oder daraus bestehen.

Mit diesen Materialien kann die Probenvorrichtung auf einfache Art und Weise unter Verwendung von bekannten Herstellungsverfahren unter Hitzeeinfluss hergestellt werden.

Insbesondere können der erste Behälter, der zweite Behälter und die Strebe das thermoplastische Polymer und/oder das Elastomer umfassen oder daraus bestehen. Die Probenvorrichtung kann im Spritzguss-, Tiefzieh- oder 3D-Druckverfahren hergestellt werden.

Die Erfindung stellt weiterhin ein Verfahren zur Herstellung einer mikrofluidischen Untersuchungsvorrichtung, insbesondere der Probenvorrichtung nach einem der vorhergehenden Ansprüche, für Untersuchungen, umfassend die folgenden Schritte bereit. Die Schritte umfassen ein Anordnen eines Einsatzes umfassend einen ersten Behälter, der an seiner Unterseite eine erste Öffnung in einer ersten Bodenebene aufweist, und einen zweiten Behälter, der an seiner Unterseite eine zweite Öffnung in einer zweiten Bodenebene aufweist, an einem Bodenelement, wobei der erste Behälter zumindest teilweise im Inneren des zweiten Behälters angeordnet ist, wobei die erste Bodenebene gegenüber der zweiten Bodenebene nach oben versetzt ist. Die Schritte umfassen weiterhin ein Einbringen eines Hydrogels in das Innere des ersten Behälters, so dass die erste Öffnung des ersten Behälters verschlossen ist.

Durch ein entsprechendes Verfahren können Teile einer Probe bzw. eines Mediums, das die Probe umgibt, getrennt werden.

Das Hydrogel kann nach dem Einbringen in das Innere des ersten Behälters eine Dicke von mindestens 0,05 mm, insbesondere mindestens 0,1 mm, insbesondere mindestens 0,5 mm, insbesondere mindestens 1 mm und/oder höchstens 5 mm, insbesondere höchstens 4,5 mm insbesondere höchstens 4 mm, insbesondere höchstens 3 mm, aufweisen. Dabei kann jede der angegebenen Untergrenzen mit jeder der angegebenen Obergrenzen kombiniert werden.

Durch die mittels des Hydrogels verschlossene erste Öffnung kann durch Diffusion bzw. Fluss aufgrund eines Druckunterschieds Flüssigkeit hindurchtreten.

Das Verfahren ist auch mit der Probenvorrichtung mit einem zweiten Behälter, der einen geschlossenen Boden aufweist, durchführbar und umfasst dann lediglich den Schritt des Einbringens eines Hydrogels in das Innere des ersten Behälters, so dass die erste Öffnung des ersten Behälters verschlossen ist.

Die vorliegende Erfindung wird anhand der nachfolgenden beispielhaften Figuren näher erläutert. Dabei zeigen:
- Fig. 1: schematisch eine Ausführungsform einer Probenvorrichtung in einer Schnittansicht,
- Fig. 2: schematisch eine weitere Ausführungsform einer Probenvorrichtung in einer Draufsicht,
- Fig. 3: schematisch die Ausführungsform der Probenvorrichtung der Fig. 2 in einer Schnittansicht entlang der Linie A-A,
- Fig. 4: schematisch eine weitere Ausführungsform einer Probenvorrichtung in einer Schnittansicht.

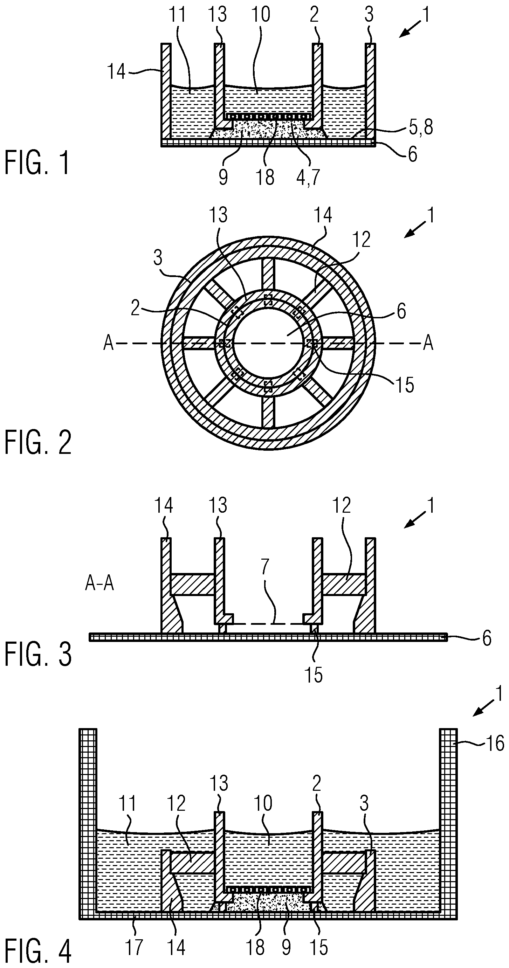

Fig. 1 bis 4 illustrieren den schematischen Aufbau von drei unterschiedlichen Ausführungsformen einer Probenvorrichtung 1.

Die Probenvorrichtung 1 in Fig. 1 umfasst einen ersten Behälter 2, der im Inneren eines zweiten Behälters 3 angeordnet ist. Der erste Behälter 2 weist an seiner Unterseite eine erste Öffnung 4 auf. Eine zweite Öffnung 5 an der Unterseite des zweiten Behälters 3 ist durch ein transparentes Bodenelement 6, beispielsweise eine Platte aus Polycarbonat, flüssigkeitsdicht verschlossen. Das Bodenelement 6 kann an dem zweiten Behälter 3 festgeklebt sein, beispielsweise über eine klebrige Oberfläche an der Unterseite des zweiten Behälters 3.

Eine erste Bodenebene 7 des ersten Behälters 2 ist gegenüber einer zweiten Bodenebene 8 des zweiten Behälters 3 nach oben versetzt angeordnet. Dadurch entsteht ein Zwischenraum, in den ein Hydrogel 9 eingebracht wird. Das Hydrogel 9 verschließt die erste Öffnung 4 und erstreckt sich bis zu dem Bodenelement 6. Das Hydrogel 9 weist in Richtung der ersten Öffnung 4 bis zu dem Bodenelement 6 eine Dicke von ca. 1 mm auf.

Wenn der erste Behälter 2 und der zweite Behälter 3 mit einer ersten Flüssigkeit 10 bzw. einer zweiten Flüssigkeit 11 wie in Figur 1 gezeigt befüllt werden, so kommunizieren die Flüssigkeiten in den Behältern über das Hydrogel 9.

Bei identischer Füllhöhe des ersten und des zweiten Behälters 3 sind die Flüssigkeiten in diffusiver Weise in Austausch. Das System ist dann druckfrei. Es resultiert kein Fluss durch das Hydrogel 9. Beinhalten die erste Flüssigkeit 10 und die zweite Flüssigkeit 11 unterschiedliche Konzentrationen eines Reagenzes, so bildet sich ein Konzentrationsgradient über das Hydrogel 9 bzw. über eine entsprechende Grenzfläche aus.

Bei unterschiedlicher Füllhöhe des ersten und des zweiten Behälters 3 entsteht ein Druckunterschied und es findet ein ausgleichender Fluss durch das Hydrogel 9 statt. Dieser druckgetriebene Fluss kann als Simulation eines interstitiellen Flusses durch ein Gewebe verwendet werden.

In jedem Fall entsteht an der Oberfläche des Hydrogels 9 eine Grenzfläche mit Kontakt zu beiden Flüssigkeiten. Nach Einbringen von biologischen Zellen 18 in den ersten Behälter 2 können diese biologischen Zellen 18 an der Grenzfläche beobachtet werden. Insbesondere ist inverse Mikroskopie durch das transparente Bodenelement 6 möglich.

Die Ausführungsform der Fig. 1 stellt eine mikrofluidische Untersuchungsvorrichtung dar.

Fig. 2 zeigt entsprechend der Fig. 1 eine Probenvorrichtung 1 mit einem ersten Behälter 2, einem zweiten Behälter 3 und einem Bodenelement 6.

Ein Querschnitt des ersten Behälters 2 ist kreisförmig ausgebildet. Der erste Behälter 2 liegt koaxial in dem zweiten Behälter 3, der ebenfalls einen kreisförmigen Querschnitt aufweist. Durch seitliche Streben 12 ist eine Seitenwand 13 des ersten Behälters 2 mit einer Seitenwand 14 des zweiten Behälters 3 verbunden. Die Streben 12 halten den ersten Behälter 2 seitlich in seiner koaxialen Position gegenüber dem zweiten Behälter 3. Die Streben 12 sind nur an bestimmten Stellen ausgebildet, beispielsweise acht Streben 12 gleichmäßig über den Kreisumfang der Geometrie verteilt, und erstrecken sich nicht auf den gesamten Umfang der Kreisform der Geometrie. Außerdem sind die Streben 12 gegenüber dem Bodenelement 6 und der ersten Bodenebene 7 nach oben versetzt angeordnet (siehe zur Verdeutlichung auch Fig. 3). Dadurch wird gewährleistet, dass eine zweite Flüssigkeit 11 von oben in den Raum zwischen dem ersten Behälter 2 und dem zweiten Behälter 3 eingefüllt werden kann und bis zu dem Bodenelement 6 gelangt, ohne durch die Streben 12 wesentlich behindert zu werden.

Außerdem wird der erste Behälter 2 durch Abstandshalter 15 in seiner vertikalen Position gegenüber dem Bodenelement 6 zusätzlich zu den Streben 12 gehalten. Die Abstandshalter 15 sind ebenfalls nur an bestimmten Stellen ausgebildet. Beispielsweise sind die Abstandshalter 15 in einer Draufsicht in radialen Verlängerungen von Streben 12 zur Mittelachse des zweiten Behälters 3 hin ausgebildet. Dadurch wird erreicht, dass die zweite Flüssigkeit 11 von unten an eine erste Öffnung 4 in einer ersten Bodenebene 7 des ersten Behälters 2 bzw. ein Hydrogel 9, das die erste Öffnung 4 verschließt, gelangt.

Fig. 3 zeigt eine beispielhafte Ausbildung der Streben 12 und Abstandshalter 15 in einer Querschnittsansicht entlang der Linie A-A in Fig. 2. Der erste Behälter 2, der zweite Behälter 3, die Streben 12 und die Abstandshalter 15 können gemeinsam einstückig ausgebildet sein.

Fig. 4 zeigt entsprechend der Fig. 2 und 3 eine Probenvorrichtung 1 umfassend einen ersten Behälter 2, einen zweiten Behälter 3, Streben 12 und Abstandshalter 15.

Darüber hinaus ist ein dritter Behälter 16 mit einem geschlossenen Boden 17 vorgesehen, in dessen Inneren der erste und der zweite Behälter 3 angeordnet sind. Eine klebrige Unterseite des zweiten Behälters 3 und eine klebrige Unterseite der Abstandshalter 15 können an dem Boden 17 des dritten Behälters 16 befestigt sein.

Zusätzlich können weitere erste Behälter 2 und zweite Behälter 3 gemäß der bisherigen Beschreibung in dem Inneren des dritten Behälters 16 angeordnet sein.

Eine Seitenwand 13 des ersten Behälters 2 endet oberhalb einer Seitenwand 14 des zweiten Behälters 3. Dadurch wird ermöglicht, dass der Raum zwischen dem ersten Behälter 2 und dem zweiten Behälter 3 mit derselben zweiten Flüssigkeit 11 wie der Raum zwischen dem zweiten Behälter 3 und dem dritten Behälter 16, nicht jedoch der Raum im Inneren des ersten Behälters 2, bis zu einer gemeinsamen Füllhöhe gefüllt wird. Dadurch wird das Volumen der zweiten Flüssigkeit 11 vergrößert. Diese zweite Flüssigkeit 11 gerät über das Hydrogel 9 in Kontakt mit biologischen Zellen 18 an der Grenzfläche zu einer ersten Flüssigkeit 10 in dem ersten Behälter 2.

Bei Verwendung mit mehreren ersten Behältern 2 und zweiten Behältern 3 in einem dritten Behälter 16 kann der Durchsatz parallelisiert werden. Es muss dann lediglich an einer Stelle die zweite Flüssigkeit 11 hinzugegeben werden, um alle Räume zwischen ersten Behältern 2 und zweiten Behältern 3 zu füllen.

Weiterhin kann eine gängige Mikrotiterplatte oder Petrischale beispielsweise 1, 6, 12, 24, 48, 96, 384, oder 1536 Vertiefungen als dritte Behälter 16 bereitstellen. Der dritte Behälter kann die Größe eines Objektträgers haben.

Einzelne Elemente der im Vorhergehenden beschriebenen Ausführungsformen können miteinander kombiniert werden. Beispielsweise können die Streben 12 und Abstandshalter 15 der Figuren 2, 3 und 4 auch in der Ausführungsform der Figur 1 vorgesehen sein. Weiterhin kann der dritte Behälter 16 der Figur 4 mit einem Bodenelement 6 der Figuren 1, 2 und 3 ausgetauscht werden und umgekehrt.

### Bezugszeichenliste:

- 1: Probenvorrichtung
- 2: erster Behälter
- 3: zweiter Behälter
- 4: erste Öffnung
- 5: zweite Öffnung
- 6: Bodenelement
- 7: erste Bodenebene
- 8: zweite Bodenebene
- 9: Hydrogel
- 10: erste Flüssigkeit
- 11: zweite Flüssigkeit
- 12: Strebe
- 13: Seitenwand des ersten Behälters
- 14: Seitenwand des zweiten Behälters
- 15: Abstandshalter
- 16: dritter Behälter
- 17: Boden

## Patentansprüche

1. Mikrofluidische Probenvorrichtung (1) umfassend:
einen ersten Behälter (2), der an seiner Unterseite eine erste Öffnung (4) in einer ersten Bodenebene (7) aufweist, und
einen zweiten Behälter (3), der an seiner Unterseite eine zweite Öffnung (5) in einer zweiten Bodenebene (8) aufweist,
wobei der erste Behälter zumindest teilweise im Inneren des zweiten Behälters angeordnet ist,
wobei der erste Behälter mit dem zweiten Behälter verbunden ist und
wobei die erste Bodenebene gegenüber der zweiten Bodenebene nach oben versetzt ist.

2. Probenvorrichtung nach Anspruch 1 weiterhin umfassend:
ein Bodenelement (6), das die zweite Öffnung verschließt.

3. Probenvorrichtung nach einem der vorhergehenden Ansprüche, weiterhin umfassend:
einen dritten Behälter (16), der einen geschlossenen Boden (17) aufweist,
wobei der erste Behälter und der zweite Behälter zumindest teilweise im Inneren des dritten Behälters angeordnet sind.

4. Mikrofluidische Probenvorrichtung umfassend:
einen ersten Behälter (2), der an seiner Unterseite eine erste Öffnung (4) in einer ersten Bodenebene (7) aufweist, und
einen zweiten Behälter (3), der einen geschlossenen Boden aufweist,
wobei der erste Behälter zumindest teilweise im Inneren des zweiten Behälters angeordnet ist,
wobei der erste Behälter mit dem zweiten Behälter verbunden ist und
wobei die erste Bodenebene gegenüber dem Boden nach oben versetzt ist.

5. Probenvorrichtung nach einem der Ansprüche 2 bis 4,
wobei das Bodenelement bzw. der Boden ein transparentes Material umfasst oder daraus besteht.

6. Probenvorrichtung nach einem der Ansprüche 2 bis 5,
wobei das Bodenelement bzw. der Boden eine Dicke von mindestens 25 µm, insbesondere mindestens 100 µm, insbesondere mindestens 500 µm, und/oder höchstens 2 mm, insbesondere höchstens 1,5 mm, insbesondere höchstens 1 mm, aufweist.

7. Probenvorrichtung nach einem der vorhergehenden Ansprüche weiterhin umfassend:
eine Strebe (12), mittels der der erste Behälter seitlich mit dem zweiten Behälter verbunden ist.

8. Probenvorrichtung nach Anspruch 7,
wobei die Strebe oberhalb der ersten Bodenebene angeordnet ist.

9. Probenvorrichtung nach einem der vorhergehenden Ansprüche weiterhin umfassend:
einen Abstandshalter (15), der an der Unterseite des ersten Behälters befestigt ist,
wobei der Abstandshalter bis zur zweiten Bodenebene bzw. bis zum Boden reicht.

10. Probenvorrichtung nach einem der vorhergehenden Ansprüche,
wobei der zweite Behälter und/oder der Abstandshalter an ihrer Unterseite eine klebrige Oberfläche aufweisen.

11. Probenvorrichtung nach einem der vorhergehenden Ansprüche weiterhin umfassend:
ein Hydrogel (9), das die erste Öffnung verschließt.

12. Probenvorrichtung nach einem der vorhergehenden Ansprüche,
wobei der erste Behälter ein Volumen von mindestens 20 µl, insbesondere mindestens 50 µl, insbesondere mindestens 100 µl, insbesondere mindestens 500 µl, und/oder höchstens 2000 µl, insbesondere höchstens 1500 µl, insbesondere höchstens 1000 µl, hat.

13. Probenvorrichtung nach einem der vorhergehenden Ansprüche,
wobei die erste Öffnung einen Durchmesser von mindestens 2 mm aufweist.

14. Probenvorrichtung nach einem der vorhergehenden Ansprüche,
wobei die Probenvorrichtung ein thermoplastisches Polymer und/oder ein Elastomer umfasst oder daraus besteht.

15. Verfahren zur Herstellung einer mikrofluidischen Untersuchungsvorrichtung, insbesondere der Probenvorrichtung (1) nach einem der vorhergehenden Ansprüche, für Untersuchungen, umfassend die folgenden Schritte:
Anordnen eines Einsatzes umfassend einen ersten Behälter (2), der an seiner Unterseite eine erste Öffnung (4) in einer ersten Bodenebene (7) aufweist, und einen zweiten Behälter (3), der an seiner Unterseite eine zweite Öffnung (5) in einer zweiten Bodenebene (8) aufweist, an einem Bodenelement (6), wobei der erste Behälter zumindest teilweise im Inneren des zweiten Behälters angeordnet ist, wobei die erste Bodenebene gegenüber der zweiten Bodenebene nach oben versetzt ist,
Einbringen eines Hydrogels (9) in das Innere des ersten Behälters, so dass die erste Öffnung des ersten Behälters verschlossen ist.
